# EUROPÄISCHE PATENTANMELDUNG

(11) **EP 1 300 163 A1**
(43) Veröffentlichungstag der Anmeldung: **09.04.2003**
(21) Anmeldenummer: 02021049.8
(22) Anmeldetag: 20.09.2002
(51) Int. Cl.: A61L 11/00, B02C 19/12, B09B 3/00

(54) **Vorrichtung zum Bearbeiten von medizinischem Abfall**

(30) Priorität: 08.10.2001 DE 20116475 U
(71) Anmelder: Salda, Luciano, 41058 Vignola (MO) (IT)
(72) Erfinder: Salda, Luciano, 41058 Vignola (MO) (IT)
(74) Vertreter: Grünecker, Kinkeldey, Stockmair & Schwanhäusser Anwaltssozietät

(57) **Zusammenfassung**

Die Erfindung betrifft eine Vorrichtung (1) zum Bearbeiten, insbesondere Zerkleinern und Sterilisieren, von medizinischem Abfall. Diese Vorrichtung besitzt einen Eingaberaum (2), einen Bearbeitungsraum, einen Auslassraum (17), einen ringförmig in sich geschlossen Druckluftkanal (10) mit verschließbaren Öffnungen, der u.a. den Eingaberaum (2) mit dem Auslassraum (17) verbindet, ein Gebläse (11) zum Erzeugen einer Luftströmung in diesem Druckluftkanal (10) sowie eine Heizung für die im Druckluftkanal (10) umlaufende Luft.
Speziell ist der Bearbeitungsraum der Vorrichtung identisch mit dem in sich geschlossenen Druckluftkanal (10), so dass dieser vollständig und mit hoher Geschwindigkeit vom Abfall durchlaufen wird.

## Beschreibung

Die Erfindung betrifft eine Vorrichtung zum Bearbeiten, insbesondere zum Sterilisieren, von medizinischem Abfall. Diese Vorrichtung besitzt einen Eingaberaum, einen Bearbeitungsraum zum Sterilisieren, einen Auslassraum, einen ringförmig in sich geschlossen Druckluftkanal mit verschließbaren Öffnungen, der u.a. den Eingaberaum mit dem Auslassraum verbindet, ein Gebläse zum Erzeugen einer Luftströmung in diesem Druckluftkanal sowie eine Heizung für die im Druckluftkanal umlaufende Luft.

Der in Krankenhäusern, Arztpraxen oder ähnlichen medizinischen Einrichtungen erzeugte Abfall weist in der Regel stark infektiöse Anteile auf und muss daher insgesamt als Sondermüll behandelt werden. Erst eine Sterilisierung ermöglicht eine kostengünstige und gefahrlose Entsorgung des medizinischen Abfalls zusammen mit sonstigen Abfällen auf einer Deponie. Solch eine Sterilisierung kann auf verschiedenen Wegen durchgeführt werden: z.B. chemisch, insbesondere nass-chemisch, durch Bestrahlung mittels Ultraviolett-Licht (UV) oder Mikrowellen, oder rein thermisch durch Erhitzen auf 200°C oder mehr für eine bestimmte Mindestdauer.
Solch ein thermisches Sterilisieren durch Ultrahocherhitzen vermeidet eine chemische Belastung des Abfalls und gewährleistet im Gegensatz zu einer UV-Bestrahlung eine gleichmäßige Erhitzung des Abfalls. Ferner hat ein rein thermisches Sterilisieren gerade bei einem hohen Anteil von Metallteilen im Abfall gegenüber Mikrowellen den Vorteil, dass keine Strahlungsabschattung stattfinden kann.

Eine Vorrichtung für eine Bearbeitung von infektiösen medizinischen Abfällen ist beispielsweise aus der Japanischen Patentschrift JP 05023657 A bekannt. Dort ist der zu sterilisierende Abfall in einer Bearbeitungskammer sowohl einer Mikrowellenbestrahlung, als auch einer heißen Luftströmung ausgesetzt, wobei diese Luftströmung über ein Gebläse, eine Heizvorrichtung und die Bearbeitungskammer zirkuliert und den Abfall aufwirbelt, während dieser durch die Mikrowellenbestrahlung sterilisiert wird.

Aufgabe der vorliegenden Erfindung ist es, eine Bearbeitungsvorrichtung für medizinischen Abfall zu schaffen, die im Unterschied zu bisher bekannten Vorrichtungen eine Sterilisierung durch Ultrahocherhitzen noch effektiver gestaltet und sie in kürzerer Zeit durchführen lässt. rung durch Ultrahocherhitzen noch effektiver gestaltet und sie in kürzerer Zeit durchführen lässt.

Diese Aufgabe wird in Verbindung mit dem Merkmal des Oberbegriffs des Anspruchs 1 dadurch gelöst, dass der gesamte Druckluftkanal den Bearbeitungsraum für den Abfall bildet, und dass der Bearbeitungsraum zusätzlich zum Zerkleinern des Abfalls dient.

Diese Ausbildung führt in Verbindung mit dem Merkmal des Anspruchs 2 dazu, dass der Abfall den Druckluftkanal während der sterilisierenden Bearbeitung mit hoher Geschwindigkeit durchläuft. Infolgedessen kollidiert der Abfall mehrfach vehement mit der Wand des Druckluftkanals, wird dabei zertrümmert und so ständig weiter in kleinere Bestandteile zerkleinert. Dadurch vergrößert sich seine resultierende Gesamt-Oberfläche, was seine Erhitzung begünstigt und damit die Sterilisierzeit verringert.

Durch die erzwungene Zertrümmerung in kleinere Bestandteile können diese Bestandteile schneller und auf höhere Geschwindigkeiten beschleunigt werden, was wiederum eine weitere Zertrümmerung begünstigt. Zudem wird wegen der Zerkleinerung die maximal zurückzulegende Eindringtiefe der Wärme bis in das Innere der Abfallbestandteile verringert. Auch dies vermindert die für die Sterilisierung benötigte Zeit und führt zu einer gleichmäßigeren Erhitzung des Abfalls.

Ferner verteilt sich der Abfall auf das gesamte Volumen des Druckluftkanals. Da dieser wegen der gleichmäßigen Erhitzung der Luft insgesamt den Bearbeitungsraum bildet, ist bei der Erfindung die mittlere Dichte des Abfalls in der Vorrichtung deutlich geringer als bei bisher bekannten Vorrichtungen zum Bearbeiten von medizinischem Abfall. Auch dies führt bei einer Sterilisierung durch Ultrahocherhitzen zu einer verringerten Bearbeitungszeit.

Da der Abfall sich die gesamte Zeit über im Druckluftkanal und damit in dem Bearbeitungsraum befindet, ist die Bearbeitungszeit sehr leicht zu kontrollieren und zu variieren, da sie der Zeit zwischen dem Erreichen der Sterilisierungstemperatur und dem Öffnen des Druckluftkanals entspricht. So kann sie z.B. bei jeder neu eingefüllten Ladung an deren jeweilige Zusammensetzung angepasst werden.

Die ringförmige Ausbildung des Druckluftkanals hat den Vorteil, vom Abfall vielmals durchlaufen zu werden. So kann dieser beliebig oft beschleunigt und weiter zertrümmert werden, ohne dass die Vorrichtung in irgendeiner Weise verändert werden müsste. Dies ist mit einem linear angelegten Verarbeitungspfad unmöglich.

Der außerhalb des Druckluftkanals liegende Eingaberaum hat verschiedene vorteilhafte Funktionen. Zum einen dient er dem Einfüllen des Abfalls in die Vorrichtung, zum anderen dient er dazu, die zu einem Bearbeitungsvorgang auf einmal eingefüllte Abfallmenge auf ein Maximalvolumen zu begrenzen. Damit bestimmt er wesentlich die während der Bearbeitung vorliegende mittlere Dichte des Abfalls im Druckluftkanal. Und schließlich kann er eine Schleuse zwischen der Außenwelt und dem Innern der Vorrichtung, speziell ihrem Bearbeitungsraum, bilden. Daher werden im Folgenden seine Öffnungen als Schleuseneinlass und Schleusenauslass bezeichnet.

Ist der Eingaberaum senkrecht über dem Druckluftkanal angeordnet und befindet sich der Schleusenauslass in seinem unteren Bereich, so kann der im Eingaberaum enthaltene Abfall ohne zusätzliche Einrichtungen allein durch sein Eigengewicht in den Druckluftkanal und damit in den Bearbeitungsraum hineinfallen.

Verschlüsse des Schleusenein- und Auslasses bieten den Vorteil, bei der Befüllung der Vorrichtung und während der Bearbeitung das Innere der Vorrichtung luftdicht gegen die Außenwelt abzudichten, so dass weder kontaminierter und nicht vollständig sterilisierter Abfall noch ein Strom beschleunigter, heißer Luft nach außen dringen können. Der Verschluss des Druckluftkanals gegenüber der Außenwelt hat zudem den Vorteil, dass ein Wärmetransport nach außen unterbunden wird und daher eine schnelle Temperaturerhöhung stattfinden kann.
Wegen des Verschlusses des Eingaberaums gegenüber dem Druckluftkanal kann ebensowenig Abfall während der Bearbeitung zurück in den Eingaberaum gelangen und sich dort in einem toten Winkel der Luftströmung festsetzen.

Vorteilhafterweise können die Verschlüsse des Schleusenein- und Auslasses durch Kugelventile gebildet werden, da diese einen effektiven und luftdichten Verschluss gewährleisten.

Je höher die Geschwindigkeit, auf die die Bestandteile des Abfalls beschleunigt werden, desto effektiver ist die aus einer Kollision resultierende Zertrümmerung. Gleichzeitig steigen jedoch die an das Gebläse gestellten Anforderungen. Vorteilhafterweise ist die Leistung des Gebläses daher so hoch, dass es im Druckluftkanal eine Luftströmung mit Geschwindigkeiten bis etwa 200 km/h erzeugen kann, ggf. auch höher. Diese Geschwindigkeiten erlauben in der Regel eine ausreichende Zertrümmerung des Abfalls. Die Schaufeln des Gebläses können dabei außer zum Beschleunigen der Luft und des Abfalls auch zum weiteren Zerkleinern des Abfalls dienen.

Damit der Abfall durch die Luftströmung auf möglichst hohe Geschwindigkeiten beschleunigt werden kann, weist der Druckluftkanal bevorzugt mindestens eine gerade Strecke auf. Entlang dieser Strecke oder Strecken finden weniger oder keine Kollisionen statt, so dass die mittlere freie Zeit zum Beschleunigen zwischen zwei Kollisionen maximiert und so die nachfolgende Aufprallgeschwindigkeit erhöht wird.

Die zwischen geraden Strecken liegenden Krümmungen des Druckluftkanals haben bevorzugt einen relativ kleinen Krümmungsradius. Auf Grund der Trägheit des beschleunigten Abfalls behält dieser in Krümmungen zunächst seine Bewegungsrichtung bei. Seine nachfolgende Kollision mit der Wand des Druckluftkanals ist dann um so stärker, je kleiner der Aufprallwinkel zwischen der Bewegungsrichtung und der Flächennormalen der Wand ist, d.h. je schärfer die Krümmung des Druckluftkanals ist.

Um die Luftgeschwindigkeit durch Düseneffekte zusätzlich zu erhöhen, kann der Druckluftkanal mindestens eine oder auch mehrere Verengungen aufweisen. Diese sind vorteilhafterweise in Abschnitten des Druckluftkanals angeordnet, nach denen mit hoher Wahrscheinlichkeit eine Kollision der Abfallteile mit Wänden oder anderen Hindernissen stattfindet, da diese Kollisionen dann wegen der höheren Aufprallgeschwindigkeit mit um so größerem Impuls erfolgen.

Das Gebläse kann sich in einer Gebläsekammer befinden, die selbst ein Bestandteil des Druckluftkanals ist. In der Gebläsekammer kann eine starke Umlenkung der Luftströmung erfolgen. In Bereich der Gebläsekammer kann ferner eine geeignete Aufprall-Einrichtung für den ankommenden Abfall vorgesehen werden, an der eine besonders starke Kollision und damit eine effektive Zertrümmerung stattfindet. Dazu ist diese Aufprall-Einrichtung bevorzugt in direkter Verlängerung des Druckluftkanals angeordnet, damit auch in direkter Verlängerung der Bewegungsrichtung des Abfalls. Die Aufprall-Einrichtung kann dabei beispielsweise aus der Nabe des Gebläses, einer Wand der Gebläsekammer oder einer zusätzlich installierten Aufprallwand gebildet werden. Unmittelbar vor der Gebläsekammer kann der Druckluftkanal als Düse eine Verengung aufweisen.

Vorteilhafterweise ist die Leistung des Gebläses regelbar und damit auch die durch das Gebläse erzeugte Geschwindigkeit der Luftströmung. So kann beim Umfüllen des Abfalls aus dem Eingaberaum in den Druckluftkanal die Luftgeschwindigkeit gerade so hoch eingestellt werden, dass der Abfall aus dem Abschnitt direkt unterhalb des Schleusenauslasses forttransportiert wird, damit der übrige Abfall ohne Stau nachfolgen kann. Zudem können so aerodynamische Effekte einen Unterdruck im Druckluftkanal gegenüber dem Eingaberaum hervorrufen, der ein Hineinsaugen des Abfalls in den Druckluftkanal bewirkt, falls der Abfall nicht schon durch sein Eigengewicht durch die Auslassöffnung fällt.
Nachdem das Gebläse während der Sterilisierung seine Maximalleistung ausübt, um möglichst hohe Luftgeschwindigkeiten zu erzielen, wird es vorteilhafterweise beim Entladevorgang wieder gedrosselt, da dann nur noch ein Transport des Abfalls zum Auslassraum angestrebt ist.

Damit die Heizelemente den Strom der Luft nicht behindern, sind sie vorteilhafterweise in oder außen an der Wand des Druckluftkanals angeordnet. Auch sie können in ihrer Leistung regelbar sein, damit die Temperatur während der Sterilisierung konstant gehalten und anschließend während des Entlade- und Befüllvorgangs gesenkt werden kann, um die weitere Behandlung des Abfalls zu erleichtern. Um die Konstanz einer bestimmten Temperatur der Luft zu gewährleisten, die bevorzugt bis zu etwa 250°C beträgt, kann die Vorrichtung einen Temperatursensor aufweisen, der die Temperatur der Luft innerhalb des Druckluftkanals misst. Zwischen dem Temperatursensor und den Heizelementen besteht dann vorteilhafterweise ein geeigneter Rückkopplungs- und Regelmechanismus.

Der Auslassraum der Vorrichtung kann einen Abschnitt des Druckluftkanals räumlich umfassen. Dadurch ergibt sich der Vorteil, dass der Druckluftkanal am oder im Auslassraum zum Entladen des Bearbeitungsraums geöffnet werden kann. Diese Öffnung ist bevorzugt so gestaltet, dass die Zirkulation der Luftströmung trotz der Öffnung aufrechterhalten bleibt. Zum Beispiel kann die Öffnung dadurch entstehen, dass ein Wandabschnitt des Druckluftkanals als Schiebehülse ausgebildet ist, die durch ihr Verschieben eine Öffnung des Druckluftkanals freigibt.

Zwar kann auch bereits aus einer derart gestalteten Öffnung der sterilisierte Abfall in den die Öffnung umfassenden Auslassraum gelangen. Deutlich effektiver und schneller geschieht dies jedoch, indem ein Ablenker vorgesehen ist, der in dem geöffneten Abschnitt des Druckluftkanals in die Luftströmung eingebracht werden kann und dazu dient, die in der Luftströmung transportierten Abfallbestandteile in den Auslassraum abzulenken, ohne dass dabei die Luftströmung abreißt.

Bevorzugt weist die Auslassöffnung des Auslassraums einen Verschluss auf, damit der Auslassraum zum Entladen der Vorrichtung eine Schleuse bildet. Dieser Verschluss kann beispielsweise als Kugelventil oder als verschließbare Klappe ausgebildet sein.

Vorteilhafterweise liegt der Druckluftkanal im Wesentlichen in einer horizontalen Ebene; denn dann kann in allen seinen Abschnitten die Beschleunigung des Abfalls gleichmäßig erfolgen, unabhängig vom Eigengewicht des Abfalls.

Andererseits kann dieses Eigengewicht zum zusätzlichen Beschleunigen gerade dadurch ausgenutzt werden, dass der Druckluftkanal einen oder mehrere vertikale Anschnitte besitzt, innerhalb dessen bzw. deren der Abfall nicht nur durch die Luftströmung, sondern auch durch die Gravitation beschleunigt wird. Am unteren Ende eines vertikalen Abschnittes hat er folglich eine höhere Geschwindigkeit und somit einen größeren Impuls beim Aufprall, was die Zertrümmerung begünstigt.

Im Folgenden wird ein vorteilhaftes Ausführungsbeispiel der Erfindung anhand einer Zeichnung dargestellt. Es zeigen:
- Fig. 1: eine Draufsicht auf ein Ausführungsbeispiel einer erfindungsgemäßen Vorrichtung zur Bearbeitung von medizinischem Abfall,
- Fig. 2: eine Ansicht des Auslassraums des Ausführungsbeispiels von Fig. 1 aus der in Fig. 1 mit II bezeichneten Richtung, wobei diese Ansicht im oberen Bereich teilweise geschnitten ist,
- Fig. 3: einen Teilvertikalschnitt durch das Ausführungsbeispiel von Fig. 1 entlang der in Fig. 1 mit III bezeichneten Schnittlinie.

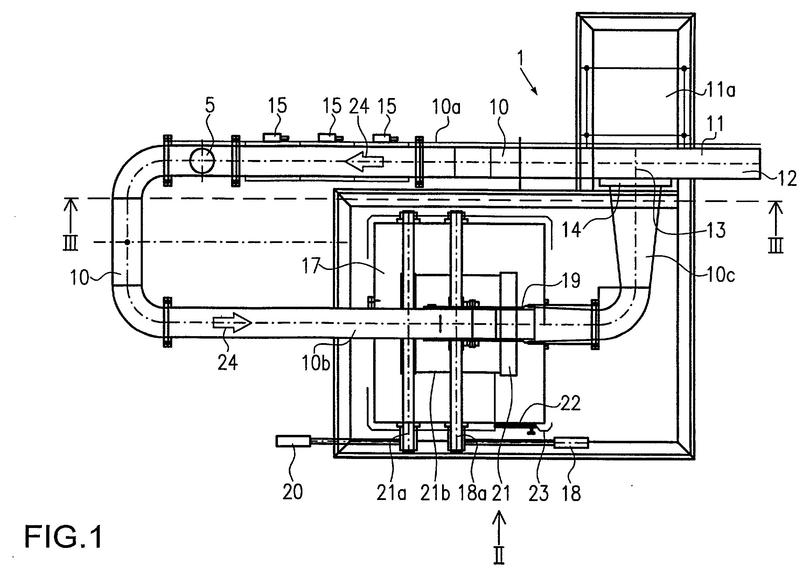

In Fig. 1 ist eine Draufsicht auf ein Ausführungsbeispiel einer erfindungsgemäßen Vorrichtung 1 gezeigt. Sie weist einen Druckluftkanal 10 auf, der identisch ist mit ihrem Bearbeitungsraum. Dieser Druckluftkanal 10 beschreibt eine geschlossene Strecke mit etwa rechteckigem Grundriss. Er liegt im Wesentlichen in einer horizontalen Ebene.

Der Druckluftkanal 10 erstreckt sich von einer Gebläsekammer 12 aus, die im Bereich einer der vier Ecken seines rechteckigen Grundrisses angeordnet ist. Von dort durchläuft er zunächst eine längere gerade Strecke, an deren Ende zum Einfüllen von zu bearbeitendem Abfall der Schleusenauslass 5 in seiner Wand liegt, speziell auf der Oberseite des Druckluftkanals 10. Der durch den Schleusenauslass 5 eingefüllte Abfall wird von der Druckluft beaufschlagt und durchläuft zusammen mit ihr den Druckluftkanal 10 in der mit Pfeilen bezeichneten Strömungsrichtung 24.

Relativ zur Strömungsrichtung 24 in dieser ersten geraden Strecke durchläuft der Druckluftkanal 10 hinter dem Schleusenauslass 5 eine erste Linkskrümmung um 90° mit relativ kleinem Krümmungsradius, gefolgt von einer kürzeren geraden Strecke und einer weiteren Linkskrümmung um 90°. Die sich daran anschließende längere gerade Strecke wird in einem Abschnitt 10b vom Auslassraum 17 umfasst, bevor eine dritte Linkskrümmung des Druckluftkanals 10 um 90° erfolgt. Auf einer an dieser dritten Linkskrümmung anschließenden kürzeren geraden Strecke 10c weitet sich der Druckluftkanal 10, bevor er wieder in die Gebläsekammer 12 mündet. Damit ist der Druckluftkanal 10 über die Gebläsekammer 12 ringförmig in sich geschlossen.

In der Gebläsekammer 12 weist die Vorrichtung 1 ein als Zentrifugalgebläse ausgebildetes Gebläse 11 zum Erzeugen einer Luftströmung mit Geschwindigkeiten bis zu etwa 200 km/h im Druckluftkanal 10 auf. Dieses Gebläse 11 weist einen Antrieb 11a und eine Nabe 13 auf. Es dient mit seinen Schaufeln nicht nur zum Beschleunigen der Luft und des Abfalls, sondern auch zur Zerkleinerung des Abfalls.

In Fig. 1 läuft die durch das Gebläse 11 erzeugte Luftströmung in Gegenuhrzeigerrichtung 24 im Druckluftkanal 10 um. Im Bereich von dessen Mündung in die Gebläsekammer 12 ist in dieser eine zusätzliche, feste Aufprall-Einrichtung 14 vorgesehen sein, die in direkter Verlängerung des Druckluftkanals 10 und damit in der Bewegungsrichtung des Abfalls liegt. In der Vorrichtung 1 ist dies z.B. die Nabe 13 des Gebläses 11. Die horizontale Ebene, in der sich der Druckluftkanal 10 im Wesentlichen befindet, liegt auf einer Höhe mit der Nabe 13 des Gebläses 11; und in seiner Mündung fluchtet die Mittellinie des Druckluftkanals 10 mit der Nabe 13.

Zum Heizen der Luftströmung im Druckluftkanal 10 sind in oder an dessen Wand 10a, insbesondere im Abschnitt zwischen der Gebläsekammer 12 und dem Schleusenauslass 5, regelbare Heizelemente 15 angeordnet. Dadurch kann der im Druckluftkanal 10 umlaufende Abfall indirekt auf Temperaturen bis zu etwa 250°C erhitzt werden.

Während des Zirkulierens und Erhitzens des Abfalls ist der vom Auslassraum 17 umfasste Abschnitt 10b des Druckluftkanals 10 verschlossen, und der Abfall durchläuft in ihm ungehindert den Auslassraum 17. Beim Entladevorgang hingegen dient der Auslassraum 17 zum Sammeln des sterilisierten Abfalls.

Zu diesem Zweck weist im Auslassraum 17 der Druckluftkanal 10 einen als Schiebehülse 19 ausgebildeten Wandabschnitt auf. Durch einen Hebel 18 als ihr Bedienelement ist diese Schiebehülse 19 in Strömungsrichtung 24 im Druckluftkanal 10 verschiebbar. Sie gibt durch ihr Verschieben eine Öffnung des Druckluftkanals 10 zum bzw. im Auslassraum 17 frei. Der in Strömungsrichtung 24 nachgeordnete Abschnitt des Druckluftkanals 10 ist entsprechend zur Aufnahme der Schiebehülse 19 geweitet. Der Hebel 18 ist um eine Achse 18a verschwenkbar.

Die Vorrichtung 1 weist im Auslassraum 17 ferner einen als gebogenen Schirm ausgebildeten Ablenker 21 auf. Dieser ist mittels eines als Hebel 20 ausgebildeten Bedienelements zwischen einer Ablenkstellung und einer Parkstellung um eine Achse 21a verschwenkbar. An der Achse 21a ist er durch Befestigungsstangen 21b angebracht.

In seiner Ablenkstellung greift der Ablenker 21 zumindest teilweise in die Luftströmung des Druckluftkanals 10 ein, d.h. er bildet ein Hindernis in der Bahn der sich im Druckluftkanal 10 bewegenden Abfallteile, ohne dass jedoch dadurch die Luftströmung abreißt. Der Ablenker 21 greift dabei durch die mittels der Schiebehülse 19 freigegebene Öffnung in den Druckluftkanal 10 hinein.

In seiner Parkstellung ist der Ablenker 21 vollständig außerhalb des Druckluftkanals 10 angeordnet, behindert also die Luftströmung nicht.

Der Auslassraum 17 weist eine Auslassöffnung 22 zur Entnahme des sterilisierte Abfalls auf. Diese Auslassöffnung 22 wiederum weist als Verschluss eine verschließbare Klappe 23 auf. Zusammen mit der Schiebehülse 19 als Verschluss des Druckluftkanals bildet der Auslassraum 17 eine Schleuse zum Entladen des Druckluftkanals 10.

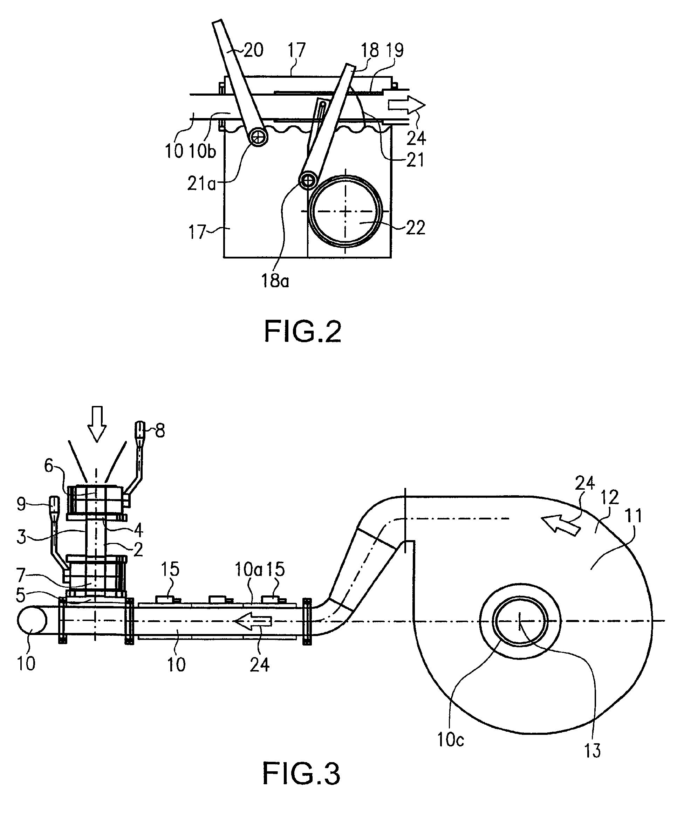

Fig. 2 zeigt eine Ansicht des Auslassraums des Ausführungsbeispiels von Fig. 1 aus der in Fig. 1 mit II bezeichneten Richtung, wobei diese Ansicht im oberen Bereich teilweise geschnitten ist, so dass der Druckluftkanal 10 zu sehen ist. Zusätzlich zu den anhand von Fig. 1 beschriebenen Merkmalen ist zu erkennen, dass der Druckluftkanal 10, insbesondere dessen Abschnitt 10b, den Auslassraum 17 in dessen oberen Bereich durchläuft. Die Auslassöffnung 22 ist im unteren Bereich des Auslassraum 17 angeordnet.

Die Achse 21a des Ablenkers 21 verläuft unterhalb des Druckluftkanals 10, ebenso wie die Achse 18a des Hebels 18 der Schiebehülse 19. Die Hebel 18 und 20 befinden sich in dieser Ansicht vor dem Auslassraum 17. In Fig. 2 ist der Ablenker 21 in seiner Ablenkstellung dargestellt. Er greift durch die mittels der Schiebehülse 19 freigegebene Öffnung des Druckluftkanals 10b in diesen hinein.

Fig. 3 zeigt einen Teilvertikalschnitt durch das Ausführungsbeispiel von Fig. 1 entlang der dort gezeigten Schnittlinie m, die den Druckluftkanal 10 an zwei Stellen schneidet. Dargestellt ist ein Abschnitt des Druckluftkanals 10, in dem die Luftströmung in Strömungsrichtung 24 verläuft. Die Gebläsekammer 12 hat in dieser Seitenansicht einen etwa kreisförmigen Querschnitt. Der Druckluftkanal 10 erstreckt sich von der Gebläsekammer 12, speziell in tangentialer Richtung von ihrer Oberseite aus. Von dort verläuft er unter einer leichten Verringerung seines Querschnitts in einer Schräge abwärts bis auf die mittlere Höhe der Gebläsekammer 12, d.h. auf die Höhe der Gebläsenabe 13.

In dieser Höhe schließt sich eine gerade und horizontale Strecke des Druckluftkanals 10 an. In oder an der Wand 10a des Druckluftkanals 10 sind entlang dieser Strecke die Heizelemente 15 angeordnet.

Am Ende der geraden Strecke schließt sich die erste Linkskrümmung des Druckluftkanals 10 an, d.h. aus der Zeichenebene heraus. Davor befindet sich oberhalb des Druckluftkanals 10 der Eingaberaum 2, der als Schleuse ausgebildet ist und über seinen Schleusenauslass 5 mit dem Druckluftkanal 10 in Verbindung steht.

Der Eingaberaum 2 dient zum Befüllen der Vorrichtung mit dem zu sterilisierenden Abfall. Er weist einen vertikal angeordneten Zylinder 3 auf, mit einem an seinem oberen Ende ausgebildeten Schleuseneinlass 4 sowie einem an seinem unteren Ende ausgebildeten Schleusenauslass 5. Dieser Schleuseneinlass 4 weist einen Verschluss 6 auf, der Schleusenauslass 5 einen Verschluss 7, wobei die Verschlüsse 6 und 7 jeweils durch Kugelventile gebildet sind. Diese weisen eine Kugel mit einer durchgehenden Bohrung auf (nicht gezeigt). Je nach Stellung der Kugel gibt die Bohrung in einer Durchgangsstellung den Weg durch das Ventil frei oder steht in einer Verschlussstellung senkrecht zu der Verbindungslinie von innen nach außen, so dass das Ventil geschlossen ist. Die Verschlüsse 6 und 7 weisen außerhalb des Eingaberaums 2 Bedienelemente 8 bzw. 9 zu ihrer Bedienung auf.

Ferner ist in Fig. 3 die sich weitende Mündung des Druckluftkanals 10c in die Gebläsekammer 12 dargestellt. Die Mittelachse des Abschnitts 10c des Druckluftkanals 10 fluchtet genau mit der Nabe 13 des Gebläses 11. Das Gebläse 11 dreht sich in der hier gezeigten Ansicht entgegen dem Uhrzeigersinn.

Weitere Ausbildungen der erfindungsgemäßen Vorrichtung sind durchaus denkbar. So können beispielsweise ein oder mehrere Mikrowellenstrahler als zusätzliche Bearbeitungseinrichtungen inkorporiert werden. Sie bieten den Vorteil, den Abfall von innen her zu erh itzen. Günstigerweise sind diese Mikrowellenstrahler dann an denjenigen Stellen angeordnet, an denen vornehmlich die Kollisionen der Abfallteile mit Hindernissen oder Wänden stattfinden; denn an diesen Stellen ist die mittlere Geschwindigkeit des Abfalls am geringsten und daher seine mittlere Aufenthaltsdauer in den bestrahlten Gebieten am längsten.

Ferner kann der Druckluftkanal eine oder mehrere Verengungen seines Querschnitts aufweisen. Darüber hinaus kann er zusätzlich zu den horizontalen Abschnitten auch einen oder mehrere vertikale Abschnitte besitzen, an denen die Gravitationskraft eine zusätzlich zur Luftströmung wirkende Beschleunigung der Abfallteile darstellt.

Im Folgenden wird anhand des in den Figuren 1-3 dargestellten Ausführungsbeispiels die Funktionsweise einer erfindungsgemäßen Vorrichtung 1 zur Bearbeitung von medizinischem Abfall erläutert. Im üblichen Betrieb laufen dabei zeitlich nacheinander ein Einfüllvorgang, ein Umfüllvorgang, ein Bearbeitungsvorgang und ein Entladevorgang ab.

Im Einfüllvorgang wirkt der Eingaberaum 2 als Schleuse zwischen der Außenwelt und dem Bearbeitungsraum 10. Zunächst wird - bei verschlossenem Schleusenauslass 5 - der Verschluss 6 des Schleuseneinlasses 4 durch das Bedienelement 8 geöffnet. Der in der Regel bereits vorher zerkleinerte, z.B. gemahlene, Abfall wird sodann von einem Bediener durch den Schleuseneinlass 4 in den Eingaberaum 2 gefüllt. Sobald der gesamte vorhandene Abfall eingefüllt oder der Eingaberaum 2 maximal gefüllt ist, wird der Schleuseneinlass 4 wieder mittels des Veschlusses 6 verschlossen. Dieser gesamte Einfüllvorgang kann unabhängig vom Betriebszustand der Bearbeitungseinrichtung efolgen, d.h. auch während einer Bearbeitung einer vorangehenden Abfall-Einheit in der Vorrichtung 1.

Das sich anschließende Umfüllen vom Eingaberaum in den Bearbeitungsraum, also der Umfüllvorgang, erfolgt erst dann, wenn der Bearbeitungsraum, also der Druckluftkanal 10, frei von Abfall des vorangehenden Durchgangs ist, und wenn der Verschluss 6 des Schleuseneinlasses 4 geschlossen ist. Für dieses Umfüllen wird zunächst der Verschluss 7 des Schleusenauslasses 5 mittels des Bedienelements 9 geöffnet. Da sich der Schleusenauslass 5 im Boden des Eingaberaums 2 befindet, fällt der Abfall auf Grund seines Eigengewichts durch den Schleusenauslass 5 in den darunterliegenden Druckluftkanal 10. Gleichzeitig wird das Gebläse 11 mit moderater Leistung betrieben, so dass der in den Druckluftkanal gelangte Abfall vom Schleusenauslass 5 forttransportiert wird. Durch die Luftströmung entsteht zudem über aerodynamische Effekte ein Unterdruck gegenüber dem Eingaberaum 2, mittels dessen der Abfall verstärkt in den Druckluftkanal 10 hineingesogen wird.

Ist der Umfüllvorgang beendet, so wird der Schleusenauslass 5 wieder durch den Verschluss 7 geschlossen und der Bearbeitungsvorgang eingeleitet. Dazu werden die Leistungen sowohl des Gebläseantriebs 11a, als auch der Heizelemente 15 erhöht, bis die Luftströmung eine Geschwindigkeit von etwa 200 km/h und eine Temperatur von etwa 250°C hat. Der im Druckluftkanal 10 enthaltene Abfall wird dadurch sowohl beschleunigt, als auch erhitzt. Er führt vehemente Kollisionen mit der Wand des Druckluftkanals 10 und der Aufprall-Einrichtung 14 aus, wird dadurch zertrümmert und durch die hohe Temperatur sterilisiert. Dieser Bearbeitungsvorgang wird für eine bestimmt Zeit, z.B. einige Minuten, aufrechterhalten, bis eine ausreichende Sterilisierung des Abfalls gewährleistet ist.

Daraufhin findet als letztes der Entladevorgang statt. Die Leistung des Gebläseantriebs 11a wird gedrosselt, die Heizelemente 15 ebenfalls gedrosselt oder ggf. ganz abgeschaltet. Die Schiebehülse 19 des Druckluftkanals 10 im Auslassraum 17 wird mittels des Bedienelements 18 in den dahinter liegenden Anschnitt des Druckluftkanals 10 hineingeschoben, so dass eine Öffnung des Druckluftkanals 10 zum Auslassraum 17 freigegeben wird.

Durch die freigegebene Öffnung, die vom Auslasraum 17 umfasst wird, wird mittels des Bedienelements 20 der Ablenker 21 (z.B. ein Ablenkschirm) in die Luftströmung eingebracht, indem er von seiner Parkstellung um die Achse 21a verschwenkt wird, bis er sich in seiner Ablenkstellung befindet. Der im Druckluftkanal 10 umlaufende Abfall trifft nun auf den Ablenker 21 auf, wird von diesem an der Fortbewegung durch den Druckluftkanal 10 gehindert und statt dessen in den Auslassraum 17 gelenkt, wo er sich sammelt.

Ist der gesamte Abfall vom Druckluftkanal 10 in den Auslassraum 17 gelangt, so wird der Entladevorgang beendet. Dazu wird die Schiebehülse 19 wieder in ihre ursprüngliche Position verschoben, so dass die Wand des Druckluftkanals 10 geschlossen ist.

Sobald damit der Entladevorgang des Bearbeitungsraums beendet ist, kann der Verschluss 23 der Auslassöffnung 22 des Auslassraums 17 geöffnet und der sterilisierte Abfall durch die Auslassöffnung 22 aus der Vorrichtung 1 entnommen werden. Gleichzeitig kann bereits ein erneutes Umfüllen von Abfall aus dem Eingaberaum 2 in den Bearbeitungsraum 10 erfolgen.

## Patentansprüche

1. Vorrichtung zum Bearbeiten von medizinischem Abfall mit einem Eingaberaum (2), einem Bearbeitungsraum zum Sterilisieren des Abfalls, einem Auslassraum (17), einem ringförmig in sich geschlossen Druckluftkanal (10) mit verschließbaren Öffnungen (5), (19), der u.a. den Eingaberaum (2) mit dem Auslassraum (17) verbindet, einem Gebläse (11) zum Erzeugen einer Luftströmung in diesem Druckluftkanal (10) und einer Heizung (15) für die im Druckluftkanal (10) umlaufende Luft, **dadurch gekennzeichnet, dass** der gesamte Druckluftkanal (10) den Bearbeitungsraum für den Abfall bildet, und dass der Bearbeitungsraum (10) zusätzlich zum Zerkleinern des Abfalls dient.

2. Vorrichtung nach Anspruch 1, **dadurch gekennzeichnet, dass** die Geschwindigkeit der Luftströmung während der Bearbeitung zwischen 100 km/h und 300 km/h beträgt, vorzugsweise etwa 200 km/h.

3. Vorrichtung nach wenigstens einem der vorangehenden Ansprüche, **dadurch gekennzeichnet, dass** der Eingaberaum (2) einen Schleuseneinlass (4) und einen Schleusenauslass (5) hat und sowohl der Schleuseneinlass (4), als auch der Schleusenauslass (5) einen Verschluss (6 bzw. 7) aufweisen.

4. Vorrichtung nach wenigstens einem der vorangehenden Ansprüche, **dadurch gekennzeichnet, dass** die Verschlüsse (6,7) von Schleuseneinlass (4) und Schleusenauslass (5) des Eingaberaums (2) durch Kugelventile gebildet werden.

5. Vorrichtung nach wenigstens einem der vorangehenden Ansprüche, **dadurch gekennzeichnet, dass** der Schleusenauslass (5) im unteren Bereich des Eingaberaums (2) angeordnet ist.

6. Vorrichtung nach wenigstens einem der vorangehenden Ansprüche, **dadurch gekennzeichnet, dass** das Gebläse (11) in einer Gebläsekammer (12) angeordnet ist, die Bestandteil des Druckluftkanals (10) ist.

7. Vorrichtung nach wenigstens einem der vorangehenden Ansprüche, **dadurch gekennzeichnet, dass** die Heizung für die im Druckluftkanal (10) umlaufende Luft aus mindestens einem Heizelement (15) besteht und die Heizelemente (15) an oder in der Wand (10a) des Druckluftkanals (10) angeordnet sind.

8. Vorrichtung nach wenigstens einem der vorangehenden Ansprüche, **dadurch gekennzeichnet, dass** die Heizelemente (15) im Abschnitt des Druckluftkanals (10) zwischen der Gebläsekammer (12) und dem Eingaberaum (2) angeordnet sind.

9. Vorrichtung nach wenigstens einem der vorangehenden Ansprüche, **dadurch gekennzeichnet, dass** die Temperatur im Druckluftkanal (10) während der Bearbeitung zwischen 180°C und 300°C beträgt, vorzugsweise etwa 250°C.

10. Vorrichtung nach wenigstens einem der vorangehenden Ansprüche, **dadurch gekennzeichnet, dass** der Druckluftkanal (10) mindestens eine gerade Strecke aufweist.

11. Vorrichtung nach wenigstens einem der vorangehenden Ansprüche, **dadurch gekennzeichnet, dass** der Druckluftkanal (10) im Bereich der Gebläsekammer (12) eine Aufprall-Einrichtung (14) aufweist.

12. Vorrichtung nach wenigstens einem der vorangehenden Ansprüche, **dadurch gekennzeichnet, dass** die Aufprall-Einrichtung (14) entweder die Nabe (13) des Gebläses (11) oder eine Wand der Gebläsekammer (12) oder eine zusätzliche Aufprallwand ist.

13. Vorrichtung nach wenigstens einem der vorangehenden Ansprüche, **dadurch gekennzeichnet, dass** der Auslassraum (17) einen Abschnitt (10b) des Druckluftkanals (10) räumlich umfasst.

14. Vorrichtung nach wenigstens einem der vorangehenden Ansprüche, **dadurch gekennzeichnet, dass** der Druckluftkanal (10) am oder im Auslassraum (17) eine verschließbare Öffnung aufweist.

15. Vorrichtung nach wenigstens einem der vorangehenden Ansprüche, **dadurch gekennzeichnet, dass** am oder im Auslassraum (17) die Wand des Druckluftkanals (10) mindestens eine verschiebbare Hülse (19) aufweist, welche durch ihr Verschieben eine Öffnung des Druckluftkanals (10) freigibt.

16. Vorrichtung nach wenigstens einem der vorangehenden Ansprüche, **dadurch gekennzeichnet, dass** der Auslassraum (17) eine Auslassöffnung (22) zur Entnahme des Abfalls aufweist.

17. Vorrichtung nach wenigstens einem der vorangehenden Ansprüche, **dadurch gekennzeichnet, dass** der Druckluftkanal (10) mindestens eine Verengung aufweist, durch die die Luftströmung beschleunigt wird.

18. Vorrichtung nach wenigstens einem der vorangehenden Ansprüche, **dadurch gekennzeichnet, dass** der Druckluftkanal (10) unmittelbar vor der Gebläsekammer (12) eine Verengung aufweist.

19. Vorrichtung nach wenigstens einem der vorangehenden Ansprüche, **dadurch gekennzeichnet, dass** der Druckluftkanal (10) hauptsächlich in einer horizontalen Ebene angeordnet ist.

20. Vorrichtung nach wenigstens einem der vorangehenden Ansprüche, **dadurch gekennzeichnet, dass** der Druckluftkanal (10) mindestens einen vertikalen Abschnitt aufweist.
